# EUROPEAN PATENT SPECIFICATION

(11) **EP 4 115 808 B1**
(45) Date of publication and mention of the grant of the patent: **01.04.2026**
(21) Application number: 22167114.2
(22) Date of filing: 07.04.2022
(51) Int. Cl.: A61B 5/257, A61B 5/282, A61B 5/291, A61B 5/296, A61N 1/04, A61B 5/021, A61B 5/145

(54) **WEARABLE DEVICE INCLUDING MOISTURE PERMEABLE STRUCTURE AND METHOD OF MANUFACTURING THE SAME**
TRAGBARE VORRICHTUNG, DIE EINE FEUCHTIGKEITSDURCHLÄSSIGE STRUKTUR UMFASST, UND VERFAHREN ZUR HERSTELLUNG DERSELBEN
DISPOSITIF PORTABLE DOTÉ D'UNE STRUCTURE PERMÉABLE À L'HUMIDITÉ ET SON PROCÉDÉ DE FABRICATION

(30) Priority: 05.07.2021 KR 20210087856
(43) Date of publication of application: 11.01.2023
(73) Proprietor: Atsens Co., Ltd., Seongnam-si Gyeonggi-do 13637 (KR)
(72) Inventor: CHAE, Deok Byeong, Seongnam-si, Gyeonggi-do (KR); PARK, Soon Keun, Yongin-si, Gyeonggi-do (KR); CHO, Seung Bum, Gwacheon-si, Gyeonggi-do (KR); JEONG, Jong Ook, Seongnam-si, Gyeonggi-do (KR)
(74) Representative: Nederlandsch Octrooibureau

(56) References cited:
- WO-A1-2017/035502
- CN-B- 108 606 788
- KR-B1- 102 208 561
- US-A1- 2015 080 697
- US-A1- 2017 333 696

## Description

### BACKGROUND

### 1. Field

The disclosure relates to a wearable device and a method of manufacturing the same, and more particularly, to a wearable device including a structure capable of effectively discharging moisture while being attached to a user's skin, and a method of manufacturing the wearable device.

### 2. Description of the Related Art

A wearable device may be attached to a user's body, such as the user's abdomen, back, shoulder, or arm, to measure biosignals such as electromyography, electrocardiogram, blood pressure, or brain waves. For example, a wearable device for measuring an electrocardiogram, whose electrodes are in contact with the user's skin, detects electrical activity in the user's heart, and measures the electrocardiogram. Due to such characteristics, the wearable device should operate while being attached to the user's body for a long time.

As described above, when the wearable device is used for a long time while being attached to the user's body, sweat is accumulated or external moisture flows into the wearable device. The sweat or the external moisture causes an impedance measurement error, which deteriorates the measurement precision. And as moisture is included in a part of the wearable device in contact with the user's skin for a long time, a mark may be left on the user's skin or an allergic reaction may be caused.

The above background art is technical information possessed by the inventor to derive the disclosure or obtained during a process of deriving the disclosure, and is not necessarily considered to be known art open to the general public prior to the filing of the disclosure.

### PRIOR ART DOCUMENTS

### PATENT DOCUMENTS

(Patent Document 1) Patent Publication No. 10-2009-0102943,
(Patent Document 2) WO 2017/035502 A1,
(Patent Document 3) KR 102208561 B1,
(Patent Document 4) US 2017/333696 A1,
(Patent Document 5) CN 108606788 B.

### SUMMARY OF THE INVENTION

According to the invention there is provided a wearable device according to claim 1 and a method of manufacturing such according to claim 6, advantageous embodiments being specified in the dependent claims 2-5.

### SUMMARY OF THE DISCLOSURE

The disclosure has been devised to solve the above technical problems and provides a wearable device including a moisture-permeable structure capable of discharging moisture from a user's skin to the outside, and a method of manufacturing the wearable device.

However, such a technical problem is an example, and the objective of disclosure to solve is not limited thereto.

Additional aspects will be set forth in part in the description which follows and, in part, will be apparent from the description, or may be learned by practice of the presented embodiments of the disclosure.

According to an embodiment of the disclosure, the wearable device, which is used by being attached to a user's skin, includes: a main body unit including a housing, a electronic unit, a battery, and a patch unit, the battery being arranged inside the housing; an electrode unit including a plurality of electrodes connected to the electronic unit; battery supplies operating power to the electronic parts; and the patch unit including one or more conductive members, the one or more conductive members being configured to electrically connect the electrode unit to the user's skin, wherein the patch unit includes a plurality of stacked layers, and a layer in contact with the user's skin includes a plurality of ventilation holes.

According to an embodiment of the disclosure, in the wearable device, the patch unit may include: a first attachment layer in direct contact with the user's skin, the first attachment layer including a plurality of first ventilation holes; an absorption layer on the first attachment layer; and a second attachment layer on the absorption layer, the second attachment layer being configured to connect the patch unit to the main body unit and including a plurality of second ventilation holes.

According to an embodiment of the disclosure, in the wearable device, when viewed from above, the plurality of first ventilation holes and the plurality of second ventilation holes may be arranged to run through.

According to an embodiment of the disclosure, in the wearable device, the patch unit may further include a current-stopping layer between the absorption layer and the second attachment layer, the current-stopping layer including a moisture-permeable material.

According to an embodiment of the disclosure, in the wearable device, the electrode unit may further include an electrode protection layer on the patch unit, the electrode protection layer including, on an inside thereof, a mounting groove corresponding to a shape of the main body unit, and including, on an outside thereof, a plurality of ventilation holes arranged in a certain pattern.

According to an embodiment of the disclosure, in the wearable device, the electrode unit may further include a shielding layer arranged under the electrode protection layer, the shielding layer including a plurality of ventilation holes arranged in a certain pattern.

According to an embodiment of the disclosure, the wearable device may further include a fixing unit having an area greater than an area of the electrode unit. The fixing unit is on the electrode unit so that at least a portion thereof is attached to the user's skin and includes a moisture-permeable material.

According to an embodiment of the disclosure, a method of manufacturing a wearable device, which is used by being attached to a user's skin, by assembling a patch unit by stacking a plurality of layers, and stacking the assembled patch unit, an electrode unit, and a main body unit, includes: forming a plurality of ventilation holes in the patch unit by blanking the plurality of stacked layers by using a press tool.

Other aspects, features, and advantages other than those described above will become apparent from the detailed description, claims and drawings for carrying out the following disclosure.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 illustrates a wearable device according to an embodiment of the disclosure;
FIG. 2 illustrates a cross-section of the wearable device taken along a line II-II of FIG. 1;
FIG. 3 is an exploded perspective view of a wearable device, according to an embodiment of the disclosure;
FIG. 4 is a top view of an electrode unit;
FIG. 5 illustrates an electrode pattern layer of an electrode unit;
FIG. 6 is a top view of a patch unit; and
FIG. 7 illustrates a wearable device according to another embodiment of the disclosure.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

The disclosure may have various modifications and various embodiments, and specific embodiments are illustrated in the drawings and are described in detail in the detailed description. However, this is not intended to limit the disclosure to particular embodiments, and it will be understood that all changes, equivalents, and substitutes that do not depart from the spirit and technical scope of the disclosure are encompassed in the disclosure. In the description of the disclosure, even though elements are illustrated in other embodiments, like reference numerals are used to refer to like elements.

Hereinafter, the disclosure will be described in detail by explaining preferred embodiments of the disclosure with reference to the attached drawings. Like reference numerals in the drawings denote like elements.

Although the terms "first," "second," etc. may be used to describe various elements, these elements should not be limited by these terms. These terms are only used to distinguish one element from another.

An expression used in the singular encompasses the expression of the plural, unless it has a clearly different meaning in the context.

It will be understood that the terms "comprise," "comprising," "include" and/or "including" as used herein specify the presence of stated features or elements but do not preclude the addition of one or more other features or elements.

Sizes of elements in the drawings may be exaggerated or reduced for convenience of explanation. In other words, because sizes and thicknesses of elements in the drawings are arbitrarily illustrated for convenience of explanation, the disclosure is not necessarily limited thereto.

In the embodiments below, the x-axis, the y-axis and the z-axis are not limited to three axes of the rectangular coordinate system, and may be interpreted in a broader sense. For example, the x-axis, the y-axis, and the z-axis may be perpendicular to one another or may represent different directions that are not perpendicular to one another.

In the case where a certain embodiment may be implemented differently, a specific process order may be performed in the order different from the described order. As an example, two processes that are successively described may be substantially simultaneously performed or performed in the order opposite to the order described.

The terms used herein are only used to describe particular embodiments and are not intended to limit the scope of the disclosure. It will be understood that the terms "comprise," "comprising," "include" and/or "including" as used herein specify the presence of stated features, numbers, steps, operations, elements, parts, and combinations thereof, but do not preclude in advance the presence or addition of one or more other features, numbers, steps, operations, elements, parts, combinations thereof.

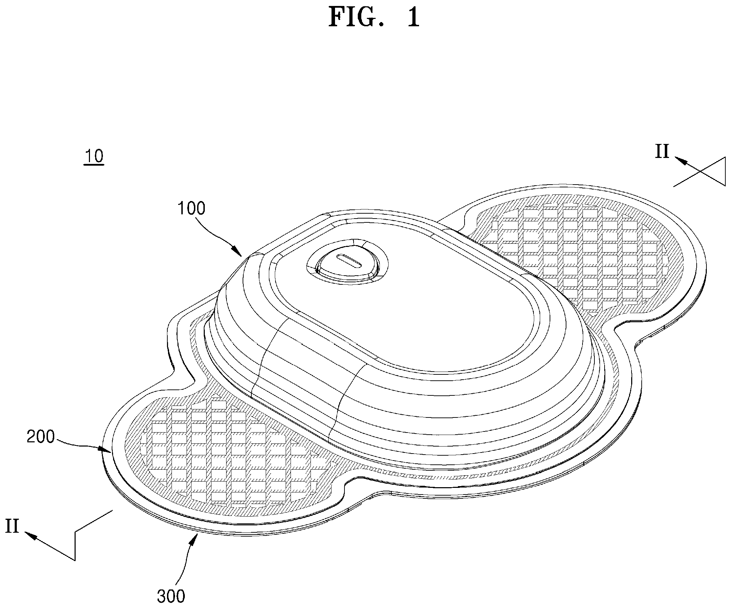

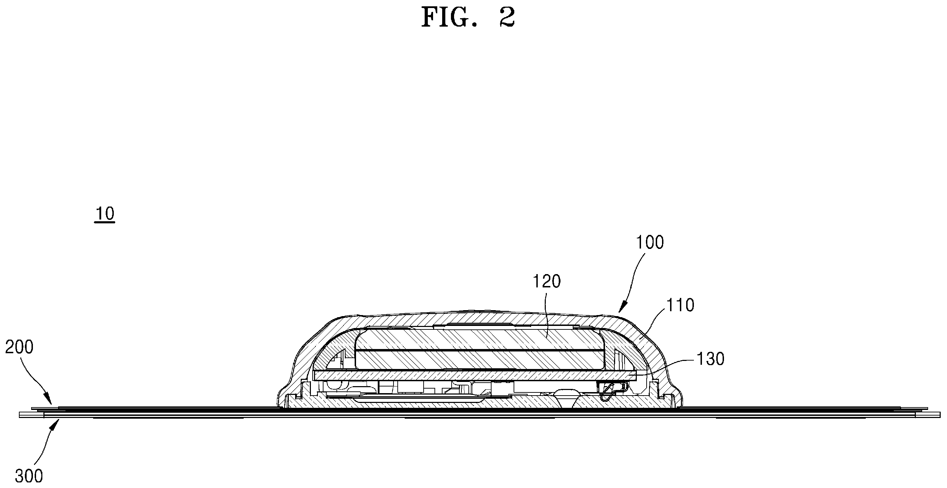

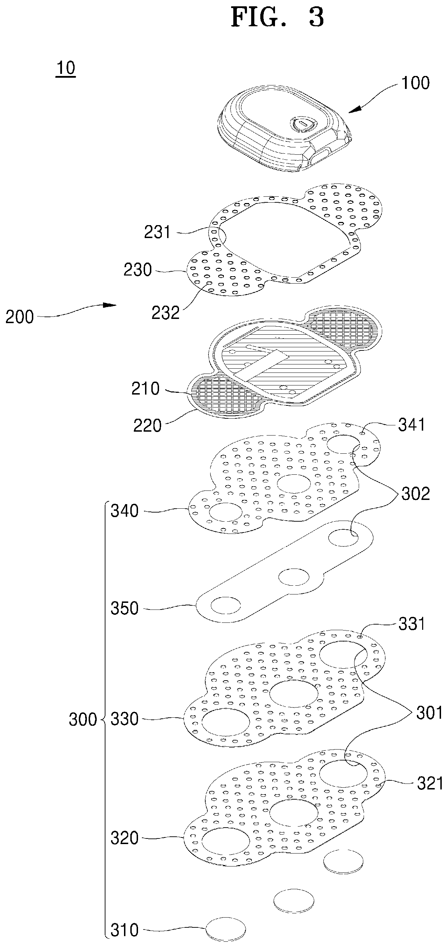

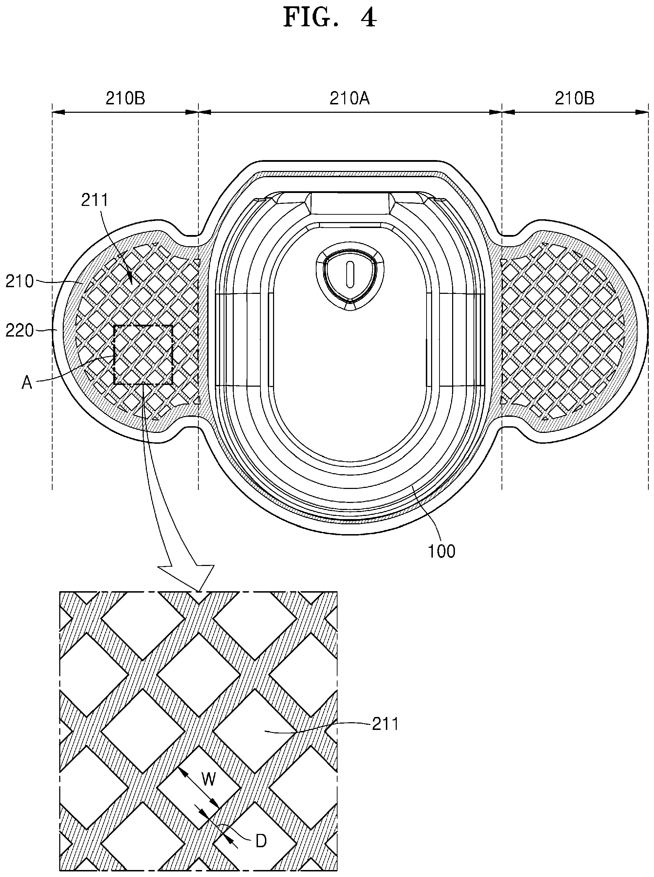

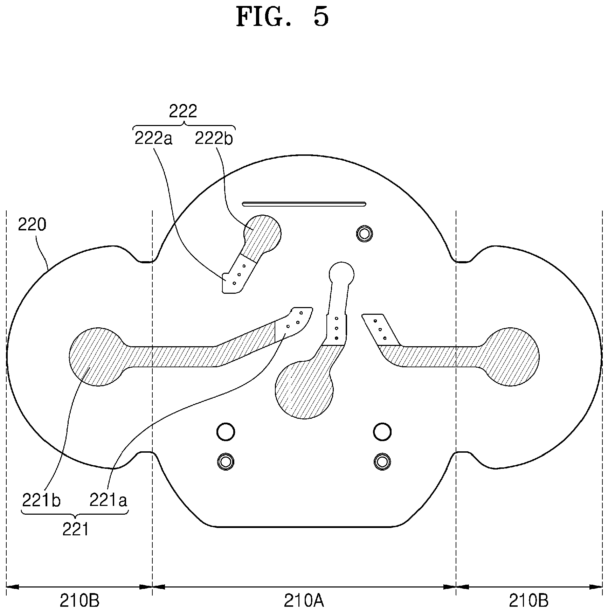

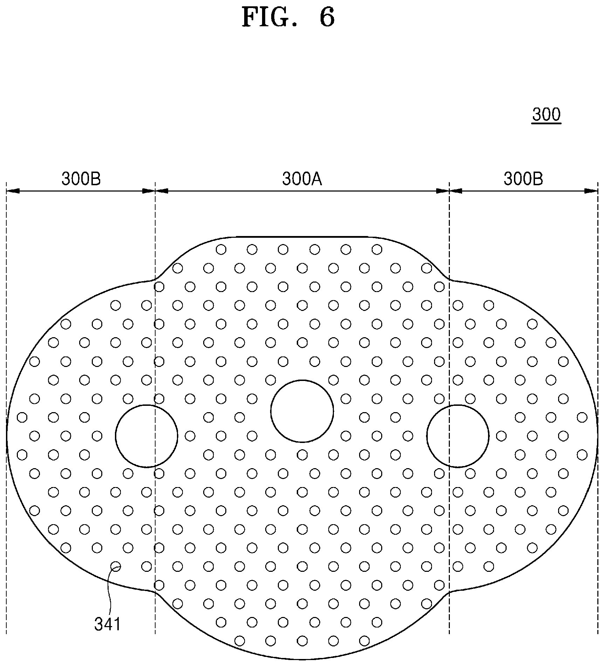

FIG. 1 illustrates a wearable device 10 according to an embodiment of the disclosure, FIG. 2 illustrates a cross-section of the wearable device 10 taken along a line II-II of FIG. 1, FIG. 3 is an exploded perspective view of the wearable device 10, according to an embodiment of the disclosure, FIG. 4 is a top view of an electrode unit 200, FIG. 5 illustrates an electrode pattern layer 220 of an electrode unit, and FIG. 6 is a top view of a patch unit 300.

According to an embodiment of the disclosure, the wearable device 10 may be an electronic device that is used by being attached to a user's back, chest, shoulder, arm, or leg. For example, according to an embodiment of the disclosure, the wearable device 10 may be a device that is attached to the user's skin and measures a biosignal such as an electrocardiogram (ECG), an electromyogram (EMG), or a blood pressure. In an embodiment, the wearable device 10 may be a device for measuring an ECG by inputting, by the electrode unit 200, a voltage generated along with a heartbeat. Alternatively, the wearable device 10 may be a device for measuring an electroencephalogram (EEG) by inputting, by the electrode unit 200, a voltage generated from cranial nerves. Alternatively, the wearable device 10 may be a device for measuring an EMG by inputting, by the electrode unit 200, a voltage generated from a skeletal muscle. Also, the wearable device 10 may be a continuous glucose monitor (CGM) or a transdermal therapeutic device using iontophoresis based on a measured biosignal such as an ECG, an EEG, or an EMG.

In an embodiment, the wearable device 10 may be in a contact switch type using a button or a touch switch type. The wearable device 10 may include a circuit for detecting the button press or detecting the touch of an object.

Referring to FIGS. 1 to 6, according to an embodiment of the disclosure, the wearable device 10 may include a main body unit 100, the electrode unit 200, and the patch unit 300.

The main body unit 100 is arranged on one side of the wearable device 10 and may include a housing 110, a battery 120, and a substrate 130. In addition, the main body unit 100 is a component for measuring an ECG while the wearable device 10 is attached to the user's body, and may include a processor, a memory, a display, an indicator, and other support structures. Hereinafter, specific components will be mainly described for convenience of description, but unless otherwise stated, components not described are not omitted or reduced in the wearable device 10.

The housing 110 protects other components of the main body unit 100 from an external shock and prevents an external foreign material from being introduced thereinto. A shape, a size, and a material of the housing 110 are not particularly limited and may be appropriately selected according to the purpose of use of the wearable device 10. In an embodiment, the housing 110 may have a dome shape including an internal space.

A button may be arranged on an upper surface of the housing 110. Although FIG. 1 illustrates that only one power button is arranged on the upper surface of the housing 110, the disclosure is not limited thereto. For example, a plurality of buttons for controlling power of the wearable device 10 and controlling an operation mode of the wearable device 10 may be arranged on the upper surface or a side surface of the housing 110.

The battery 120 and the substrate 130 may be arranged in the internal space of the housing 110. For example, as illustrated in FIG. 2, the substrate 130 may be supported by a support structure arranged in the internal space of the housing 110, and the battery 120 may be arranged on the substrate 130. Also, an electronic unit including a signal amplification circuit, a signal processing circuit, a control circuit, a processor, and a memory, which are necessary for the wearable device 10 to operate, may be arranged on the substrate 130.

In an embodiment, an antistatic paint may be coated on the inside of the main body unit 100. For example, an antistatic paint is coated or applied to the inside of the housing 110, so that external static electricity may be prevented from flowing into the main body unit 100 through the housing 110.

The electrode unit 200 may be arranged on one side of the main body unit 100 and may include a sensing electrode 221 connected to the main body unit 100. For example, the electrode unit 200 may be connected to the substrate 130 of the main body unit 100, and the sensing electrode 221 may measure a potential appearing in relation to an electrogram on the body surface or apply a current to inject an electrolyte drug into the user's body through his or her skin or mucous membrane. Also, the sensing electrode 221 may measure an impedance between electrodes.

In an embodiment, the electrode unit 200 may include a shielding layer 210 and the electrode pattern layer 220.

The shielding layer 210 may include a conductive material such as a metal, and may be arranged on an upper surface of the electrode unit 200 to prevent an abnormal potential such as external static electricity from being applied to the sensing electrode 221 and affecting a measurement signal. In an embodiment, the shielding layer 210 may be arranged to cover the electrode pattern layer 220 printed on a rear surface of the electrode unit 200.

In an embodiment, the shielding layer 210 may include a first shielding region 210A and a second shielding region 210B. In more detail, as illustrated in FIGS. 3 and 4, the first shielding region 210A may be arranged at the center of the shielding layer 210 so that the main body unit 100 is arranged inside the first shielding region 210A. Also, when viewed from above, the first shielding region 210A may be spaced apart from an edge of the main body unit 100 by a certain distance, and the main body unit 100 may be arranged inside the first shielding region 210A.

In an embodiment, when viewed from above, a connection portion of the sensing electrode 221 and the main body unit 100 may be arranged inside the first shielding region 210A. That is, the first shielding region 210A may cover a first sensing electrode terminal 221a of the sensing electrode 221, which will be described below. Also, the first shielding region 210A may cover at least one of a plurality of conductive members 310.

The second shielding region 210B may extend from both sides of the first shielding region 210A. When viewed from above, at least one conductive member 310 may be arranged inside the second shielding region 210B. That is, the second shielding region 210B may over at least one of the plurality of conductive members 310.

In an embodiment, the shielding layer 210 may float from the main body unit 100. That is, the shielding layer 210 and the main body unit 100 may maintain a state not electrically connected to each other. Accordingly, the influence of external static electricity on the main body unit 100 while flowing through the shielding layer 210 may be minimized.

In an embodiment, the shielding layer 210 may include a plurality of third ventilation holes 211 arranged in a certain pattern. For example, as illustrated in FIG. 4, when the shielding layer 210 is viewed from the top, the plurality of third ventilation holes 211 may be arranged on both sides of the shielding layer 210. That is, the third ventilation holes 211 may be arranged in the second shielding region 210B.

Accordingly, while the wearable device 10 is attached to the user's body, moisture generated from his or her skin may not flow into the main body unit 100 and may be discharged to the outside through the plurality of third ventilation holes 211.

In an embodiment, a width of a third ventilation hole 211 may be greater than a distance between adjacent third ventilation holes 211. In more detail, as illustrated in FIG. 4, a width W of the third ventilation hole 211 may be greater than a distance D between adjacent third ventilation holes 211.

Accordingly, the width of the third ventilation hole 211 may be sufficiently secured to increase moisture permeability, and static electricity applied from the outside may also flow to the outside through a ground electrode 222 of the electrode pattern layer 220, which will be described below, through a region between the third ventilation holes 211. Therefore, both the moisture permeability and an effect of preventing measurement noise caused by static electricity may be achieved.

Although FIG. 4 illustrates that the plurality of third ventilation holes 211 form a mesh structure, the disclosure is not limited thereto. For example, the plurality of third ventilation holes 211 may each have a circular shape, a polygonal shape, or a shape in which a straight line and a curved line are mixed, and may have different sizes and shapes.

In an embodiment, the shielding layer 210 may be printed on the upper surface of the electrode unit 200 with an inductive ink.

The shielding layer 210 is in a state in which a certain potential is applied through a separate circuit separated from the main body unit 100 to prevent external static electricity from flowing into the main body unit 100.

The electrode pattern layer 220 may be arranged on one surface of the electrode unit 200, for example, on the other surface of the electrode unit 200, which is a surface opposite to the shielding layer 210. For example, as illustrated in FIG. 5, the electrode pattern layer 220 may be printed on the rear surface of the electrode unit 200 and may include a plurality of sensing electrodes 221.

Although FIG. 5 illustrates that three sensing electrodes 221 are respectively arranged at the center and both sides of the electrode pattern layer 220 in a longitudinal direction, four or more sensing electrodes 221 or two or less sensing electrodes 221 may be present, and locations of the sensing electrodes 221 are not particularly limited. However, hereinafter, a case in which the wearable device 10 includes three sensing electrodes 221 will be mainly described for convenience of description.

Each of the sensing electrodes 221 may include a first sensing electrode terminal 221a connected to the main body unit 100 (in more detail, connected to the substrate 130 of the main body unit 100) and a second electrode terminal 221b connected to the conductive member 310 of the patch unit 300, which will be described below.

In an embodiment, the first sensing electrode terminal 221a of the sensing electrode 221 may be arranged at the center of the electrode pattern layer 220 in the longitudinal direction, and one second electrode terminal 221b may be arranged at each of the center and both sides of the electrode pattern layer 220.

In an embodiment, the electrode pattern layer 220 is in contact with the user's skin and is connected to the shielding layer 210, so that external static electricity may flow into the user's skin. In more detail, as illustrated in FIG. 5, the electrode pattern layer 220 may further include the ground electrode 222 in addition to the sensing electrodes 221. The ground electrode 222 may be in contact with the user's skin and may include a first ground electrode terminal 222a connected to the shielding layer 210 and a second ground electrode terminal 222b connected to the conductive member 310. Also, the ground electrode 222 may not be electrically connected to the main body unit 100 or the first ground electrode terminal 222a may be omitted.

As described above, the electrode pattern layer 220 is connected to the shielding layer 210 and the user's skin so that the user's skin may be used as a kind of ground. Accordingly, when static electricity is generated, energy of the static electricity transmitted from the shielding layer 210 may be grounded to the user's skin. Also, because the ground electrode 222 is not connected to the main body unit 100, external static electricity may be prevented from flowing into the main body unit 100.

The electrode unit 200 may further include an electrode protection layer 230. In addition to the shielding layer 210, the electrode protection layer 230 may block external static electricity to prevent static electricity from affecting the measurement accuracy of the wearable device 10. For example, as illustrated in FIG. 2, the electrode protection layer 230 may be arranged on the upper surface of the electrode unit 200, that is, on the shielding layer 210.

In an embodiment, the electrode unit 200 may include a conductor (e.g., a structure printed with a metal paint or a metal body), and a portion of the electrode unit 200 may not be electrically connected to the main body unit 100.

In an embodiment, the electrode protection layer 230 may have an area greater than those of other components of the electrode unit 200. In more detail, because the electrode protection layer 230 has an area greater than those of the shielding layer 210 and the electrode pattern layer 220, a portion of the wearable device 10 may be in contact with the user's body while the wearable device 10 is attached to the user's body.

Such a configuration may prevent external static electricity from affecting the main body unit 100 and cause static electricity to be applied to the user's body by using the user's body as a kind of ground electrode.

In an embodiment, the electrode protection layer 230 may include, on the inside thereof, a mounting groove 231 corresponding to a shape of the main body unit 100 and may include, on the outside thereof, a plurality of fourth ventilation holes 232 arranged in a certain pattern.

In more detail, as illustrated in FIGS. 1 and 2, when the electrode protection layer 230 includes the mounting groove 231 on the inside thereof and is arranged on the electrode unit 200, the main body unit 100 may be positioned inside the mounting groove 231. The mounting groove 231 may be positioned at the center of the electrode protection layer 230 in a longitudinal direction. Also, the plurality of fourth ventilation holes 232 may be arranged on both sides of the mounting groove 231, respectively.

In an embodiment, the plurality of fourth ventilation holes 232 may be arranged in the same pattern as the plurality of third ventilation holes 211. In this case, the same pattern means that sizes of a plurality of ventilation holes and distances therebetween may be slightly different from each other but shapes of the plurality of ventilation holes are the same as rectangular, square, rhombus, or circular shapes.

In an embodiment, the plurality of fourth ventilation holes 232 may be arranged to correspond to the plurality of third ventilation holes 211. That is, the plurality of fourth ventilation holes 232 may have the same size, shape, and location as the plurality of third ventilation holes 211. Also, the plurality of fourth ventilation holes 232 may have irregular shapes with permeability.

Accordingly, even though moisture is generated because the wearable device 10 is worn for a long time, moisture introduced into the electrode unit 200 through the patch unit 300 may be smoothly discharged to the outside through the third ventilation holes 211 and/or the fourth ventilation holes 232.

The patch unit 300 may be arranged under the electrode unit 200 and may be in direct contact with the user's skin to inject a drug into the user's skin.

In an embodiment, the patch unit 300 may include one or more conductive members 310 for electrically connecting the electrode unit 200 to the user's skin, and may include multiple layers including a plurality of ventilation holes.

In an embodiment, the patch unit 300 may include a main region 300A and a pair of sub-regions 300B extending from both sides of the main region 300A, respectively. For example, as illustrated in FIG. 6, the main region 300A may be arranged at the center of the patch unit 300 in a longitudinal direction, and the sub-regions 300B may be arranged on both sides of the main region 300A, respectively.

The conductive members 310 are members in direct contact with the user's skin while the wearable device 10 is attached to the user's skin, and may be electrically connected to the electrode unit 200. For example, each of the conductive members 310 may include a biocompatible material and may be hydrogel.

In an embodiment, the conductive members 310 may be in direct contact with the user's skin while being inserted into the patch unit 300 through mounting holes 301 provided in each of the main region 300A and the sub-regions 300B of the patch unit 300 by one. The mounting holes 301 may be formed through at least one of a plurality of layers of the patch unit 300 in a height direction. For example, the mounting holes 301 may be formed through a first attachment layer 320 and an absorption layer 330 of the patch unit 300, which will be described below. In addition, the conductive members 310 may be in contact with the electrode unit 200 while being inserted into the mounting holes 301.

In an embodiment, the conductive members 310 may be in contact with the electrode unit 200 while being inserted into the patch unit 300 through contact holes 302 provided in each of the main region 300A and the sub-regions 300B of the patch unit 300 by one. The contact holes 302 may be formed through at least one of the plurality of layers of the patch unit 300 in the height direction. For example, the contact holes 302 may be formed through a second attachment layer 340 and a current-stopping layer 350 of the patch unit 300, which will be described below. In addition, the conductive members 310 may be in contact with the electrode unit 200 through the contact holes 302 while being inserted into the mounting holes 301.

In an embodiment, ventilation holes of the patch unit 300 may be arranged in at least one of the main region 300A and the sub-regions 300B except for a region in which the mounting holes 301 and/or the contact holes 302 are formed.

In an embodiment, the patch unit 300 may include the first attachment layer 320, the absorption layer 330, the second attachment layer 340, and the current-stopping layer 350.

The first attachment layer 320 is a member in direct contact with the user's skin and may be an adhesive member for fixing the patch unit 300 to the user's skin. For example, the first attachment layer 320 is a tape including a biocompatible material and may be a silicon tape.

In an embodiment, the first attachment layer 320 may include a plurality of first ventilation holes 321. For example, as illustrated in FIG. 3, the first ventilation holes 321 may be randomly arranged on the entire surface of the first attachment layer 320 in a remaining region except for the mounting holes 301. Accordingly, sweat generated on the user's skin may be discharged to the outside through the first attachment layer 320 without staying in the first attachment layer 320 for a long time.

The absorption layer 330 may be arranged on the first attachment layer 320 and may include a material capable of retaining moisture so that the sweat generated on the user's skin or external moisture does not flow into the main body unit 100 or the electrode unit 200. In an embodiment, the absorption layer 330 may be a fibrous layer, and more particularly, may include a non-woven fabric and may re-discharge retained moisture.

The second attachment layer 340 is a member for attaching the patch unit 300 to the electrode unit 200, may be arranged on the absorption layer 330 to connect the electrode unit 200 to the patch unit 300, and may include a plurality of second ventilation holes 341. An adhesive material may be coated on both sides of the second attachment layer 340, and the second attachment layer 340 may be a double-sided tape.

In an embodiment, the plurality of second ventilation holes 341 may be arranged to communicate with the plurality of first ventilation holes 321 in a height direction. That is, the plurality of second ventilation holes 341 may be arranged at positions corresponding to the plurality of first ventilation holes 321. Alternatively, the plurality of second ventilation holes 341 may be arranged in the same pattern as the plurality of first ventilation holes 321. Alternatively, the plurality of second ventilation holes 341 may be arranged with the same size, shape, and number as the plurality of first ventilation holes 321.

The patch unit 300 may further include current-stopping layer 350.

As illustrated in FIG. 3, the current-stopping layer 350 may be between the absorption layer 330 and the second attachment layer 340 and may prevent fine current application between other members of the wearable device 10 or prevent fine current application between the wearable device 10 and the user's skin.

In an embodiment, the current-stopping layer 350 may include a moisture-permeable material and may be a moisture-permeable polyurethane tape.

In an embodiment, the current-stopping layer 350 may also include ventilation holes, and the ventilation holes may be arranged to communicate with ventilation holes of the first attachment layer 320, the absorption layer 330, and the second attachment layer 340. For example, the ventilation holes of the current-stopping layer 350 may be arranged with the same number, pattern, and shape as the ventilation holes of the first attachment layer 320, the absorption layer 330, and the second attachment layer 340. Although it has been described that the patch unit 300 includes a plurality of layers, the patch unit 300 may be simplified by using the same function or different materials.

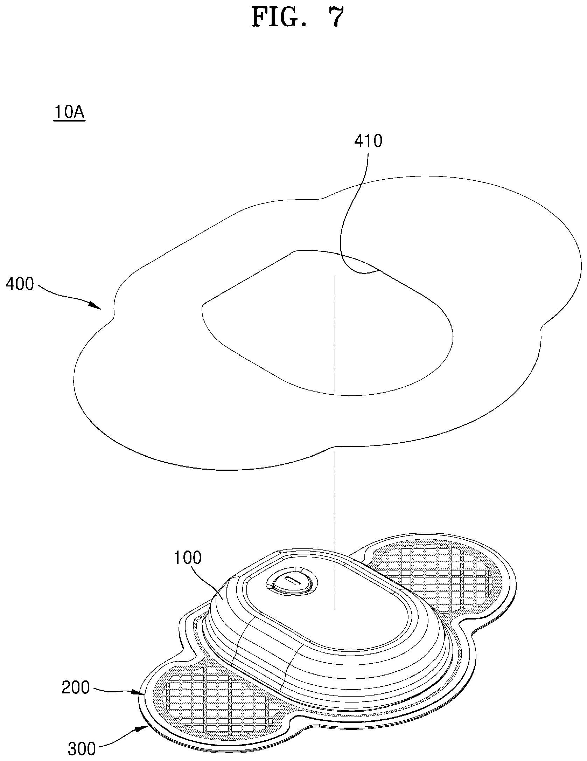

FIG. 7 illustrates a wearable device 10A according to another embodiment of the disclosure.

According to the present embodiment, the wearable device 10A may further include a fixing unit 400.

The fixing unit 400 includes an accommodation groove 410 in which the main body unit 100 is accommodated, and both side regions of the accommodation groove 410 covers the upper surface of the electrode unit 200.

In an embodiment, the fixing unit 400 may have an area greater than those of the electrode unit 200 and/or the patch unit 300. In more detail, as illustrated in FIG. 7, at least a portion of the fixing unit 400 may protrude to the outside of the electrode unit 200 and/or the patch unit 300 to adhere to the user's skin while the fixing unit 400 is arranged on the electrode unit 200.

In an embodiment, the fixing unit 400 may include a moisture-permeable material. For example, the fixing unit 400 may include a material having moisture directionality, such as a moisture-permeable polyurethane tape or Gore-Tex. Also, the fixing unit 400 may include a waterproof material. Accordingly, the fixing unit 400 may prevent external moisture from flowing into the inside of the wearable device 10A (waterproofness) and may discharge internal moisture to the outside (moisture permeability).

Hereinafter, a method of manufacturing a wearable device, according to an embodiment of the disclosure, will be described.

According to an embodiment of the disclosure, the method of manufacturing the wearable device may manufacture a wearable device 10 used by being attached to a user's skin, by assembling a patch unit 300 by stacking a plurality of layers, and stacking the assembled patch unit 300, an electrode unit 200, and a main body unit 100. Also, according to an embodiment of the disclosure, the method of manufacturing the wearable device may form a plurality of ventilation holes in the patch unit 300 by blanking the plurality of stacked layers by using a press tool.

First, the patch unit 300 is assembled by stacking the plurality of layers. For example, the patch unit 300 is assembled by stacking a first attachment layer 320, an absorption layer 330, a second attachment layer 340, and a current-stopping layer 350.

Next, the electrode unit 200 is assembled by assembling and stacking a plurality of members in a similar manner. For example, the electrode unit 200 may be assembled by forming a shielding layer 210 and an electrode pattern layer 220 in the electrode unit 200, and then arranging an electrode protection layer 230 on the shielding layer 210. However, an order of assembling the electrode unit 200 and the patch unit 300 is not particularly limited, and the electrode unit 200 may be assembled first or the electrode unit 200 and the patch unit 300 may be assembled simultaneously.

Next, ventilation holes are formed in the patch unit 300 by performing a blanking process by using the press tool. For example, the plurality of ventilation holes may be formed in the patch unit 300 by blanking the patch unit 300, in which the plurality of layers, such as the first attachment layer 320, the absorption layer 330, the second attachment layer 340, and the current-stopping layer 350, are stacked, by using the press tool.

Next, the wearable device 10 is manufactured by stacking the blanked patch unit 300, the electrode unit 200, and the main body unit 100.

In an embodiment, before the electrode unit 200 is assembled, ventilation holes are formed by blanking the shielding layer 210 and the electrode protection layer 230 by using the press tool, and then, the electrode unit 200 may be assembled. In this case, the shielding layer 210 and the electrode protection layer 230 may be blanked while the shielding layer 210 and the electrode protection layer 230 are stacked, or the shielding layer 210 and the electrode protection layer 230 may be blanked separately.

In another embodiment, when ventilation holes having different shapes and patterns are to be formed in the plurality of layers of the patch unit 300, a blanking process may be performed on each member first before the patch unit 300 is stacked. That is, a blanking process may be performed by using different press tools for each of the first attachment layer 320, the absorption layer 330, the second attachment layer 340, and the current-stopping layer 350 of the patch unit 300. Next, the patch unit 300 may be assembled by stacking the first attachment layer 320, the absorption layer 330, the second attachment layer 340, and the current-stopping layer 350.

According to an embodiment of the disclosure, the wearable device has a structure for preventing noise caused by static electricity, the structure preventing external static electricity from affecting a main body unit, and thus, the measurement accuracy of the wearable device may be prevented from deteriorating due to external static electricity.

According to an embodiment of the disclosure, in the wearable device and the method of manufacturing the same, at least one of an electrode unit and a patch unit includes a moisture-permeable material or includes ventilation holes, so that the wearable device may smoothly discharge, to the outside, moisture generated as the wearable device is used for a long time by being attached to a user's body.

As described above, the disclosure has been described with reference to the embodiment illustrated in the drawings, but this is merely an example. Those of ordinary skill in the art will fully understand that various modifications and other equivalent embodiments can be made from the embodiments. The scope of protection is determined by the appended claims.

Specific technical descriptions in the embodiments are embodiments and do not limit the technical scope of the embodiments. In order to concisely and clearly describe the disclosure, descriptions of general techniques and configurations of the related art may be omitted. Also, connections or connection members of lines between elements illustrated in the drawings are examples of functional connections and/or physical or circuit connections, and may be represented by various alternative or additional functional connections, physical connections, or circuit connections in an actual device. In addition, unless specifically stated as "essential" or "importantly", an element may not be a necessary element for the application of the disclosure.

The term "above" or similar referring expressions used in the description may refer to both the singular and plural expressions unless otherwise specified. Also, when a range is described in the embodiments, it means that embodiments to which individual values belonging to the range are applied are also included (unless otherwise stated), it is the same as each individual value constituting the range is described in the detailed description of the disclosure. Moreover, steps or operations constituting the method according to the embodiments may be performed in an appropriate order, if the order is explicitly stated or unless otherwise stated. The embodiments are not necessarily limited according to the order of the description of the steps or operations. All examples or illustrative terms (e.g., etc.) in the embodiments are merely used to describe the embodiments in detail. In addition, those of ordinary skill in the art will appreciate that various modifications, combinations, and changes can be made.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made.

## Claims

1. A wearable device (10) configured to be used by being attached to a user's skin, the wearable device (10) comprising:
a main body unit (100) including a housing (110) and a battery (120), the battery (120) being arranged inside the housing (110);
an electrode unit (200) including a plurality of electrodes (221) connected to the battery (120); and
a patch unit (300) including one or more conductive members (310), the one or more conductive members (310) being configured to electrically connect the electrode unit (200) to the user's skin, wherein the patch unit (100) includes a plurality of stacked layers, and a layer in contact with the user's skin includes a plurality of ventilation holes,
wherein the electrode unit (200) further includes an electrode protection layer (230) on the patch unit (300), the electrode protection layer (230) including, on an inside in the lengthwise thereof, a mounting groove (231) corresponding to a shape of the main body unit (100), and including, on an outside in the lengthwise thereof, a plurality of ventilation holes (232) arranged in a certain pattern,
wherein the electrode unit (200) further includes a shielding layer (210) arranged under the electrode protection layer (230), the shielding layer (210) including a plurality of ventilation holes (211) arranged in a certain pattern,
to which shielding layer a certain potential is applied through a separate circuit separated from the main body unit (100), and
wherein the ventilation holes of electrode protection layer (232) are arranged in the same pattern as the plurality of ventilation holes (211) of the shielding layer (210), the shapes of the plurality of ventilation holes are rectangular, and a width (W) of a ventilation hole is greater than a distance (D) between adjacent ventilation holes.

2. The wearable device (10) of claim 1, wherein the patch unit (300) includes:
a first attachment layer (320) in direct contact with the user's skin, the first attachment layer (320) including a plurality of first ventilation holes (321);
an absorption layer (330) on the first attachment layer (320); and
a second attachment layer (340) on the absorption layer (330), the second attachment layer (340) being configured to connect the patch unit (200) to the main body unit (100) and including a plurality of second ventilation holes (341).

3. The wearable device (10) of claim 2, wherein, the plurality of first ventilation holes (321) are arranged at positions corresponding to the plurality of second ventilation holes (341).

4. The wearable device (10) of claim 2, wherein the patch unit (200) further includes a current-stopping layer (350) between the absorption layer (330) and the second attachment layer (340), the current-stopping layer (350) including a moisture-permeable material.

5. The wearable device (10) of claim 1, further comprising a fixing unit (400) having an area greater than an area of the electrode unit (100), the fixing unit (400) being on the electrode unit (200) so that at least a portion thereof is attached to the user's skin, and including a moisture-permeable material.

6. A method of manufacturing the wearable device (10) of any one of claims 1 to 5, the method comprising:
assembling the patch unit (300) by stacking the plurality of layers,
stacking the assembled patch unit (300), the electrode unit (200) and the main body unit (100), and
forming the plurality of ventilation holes in the patch unit (300) by blanking the plurality of stacked layers by using a press tool.

## Patentansprüche

1. Tragbare Vorrichtung (10), die zur Verwendung durch Anbringen an der Haut eines Benutzers konfiguriert ist, wobei die tragbare Vorrichtung (10) Folgendes umfasst:
eine Hauptkörpereinheit (100) mit einem Gehäuse (110) und einer Batterie (120), wobei die Batterie (120) innerhalb des Gehäuses (110) angeordnet ist;
eine Elektrodeneinheit (200) mit einer Vielzahl von Elektroden (221), die mit der Batterie (120) verbunden sind; und
eine Patch-Einheit (300) mit einem oder mehreren leitfähigen Elementen (310), wobei das eine oder die mehreren leitfähigen Elemente (310) so konfiguriert sind, dass sie die Elektrodeneinheit (200) mit der Haut des Benutzers verbinden, wobei die Patch-Einheit (100) eine Vielzahl von übereinanderliegenden Schichten aufweist und eine Schicht, die mit der Haut des Benutzers in Kontakt steht, eine Vielzahl von Belüftungslöchern aufweist,
wobei die Elektrodeneinheit (200) ferner eine Elektrodenschutzschicht (230) auf der Patch-Einheit (300) aufweist, wobei die Elektrodenschutzschicht (230) auf einer Innenseite in Längsrichtung eine Befestigungsnut (231) aufweist, die einer Form der Hauptkörpereinheit (100) entspricht, und auf einer Außenseite in Längsrichtung eine Vielzahl von Belüftungslöchern (232) aufweist, die in einem bestimmten Muster angeordnet sind,
wobei die Elektrodeneinheit (200) ferner eine Abschirmschicht (210) aufweist, die unter der Elektrodenschutzschicht (230) angeordnet ist, wobei die Abschirmschicht (210) eine Vielzahl von Belüftungslöchern (211) aufweist, die in einem bestimmten Muster angeordnet sind, wobei an diese Abschirmschicht über einen von der Hauptkörpereinheit (100) getrennten separaten Stromkreis ein bestimmtes Potential angelegt wird, und
wobei die Belüftungslöcher der Elektrodenschutzschicht (232) in demselben Muster wie die Vielzahl von Belüftungslöchern (211) der Abschirmschicht (210) angeordnet sind, die Formen der Vielzahl von Belüftungslöchern rechteckig sind und eine Breite (W) eines Belüftungslochs größer als ein Abstand (D) zwischen benachbarten Belüftungslöchern ist.

2. Tragbare Vorrichtung (10) nach Anspruch 1, wobei die Patch-Einheit (300) Folgendes umfasst:
eine erste Befestigungsschicht (320) in direktem Kontakt mit der Haut des Benutzers, wobei die erste Befestigungsschicht (320) eine Vielzahl von ersten Belüftungslöchern (321) aufweist;
eine Absorptionsschicht (330) auf der ersten Befestigungsschicht (320); und
eine zweite Befestigungsschicht (340) auf der Absorptionsschicht (330), wobei die zweite Befestigungsschicht (340) so konfiguriert ist, dass sie die Patch-Einheit (200) mit der Hauptkörpereinheit (100) verbindet, und eine Vielzahl von zweiten Belüftungslöchern (341) aufweist.

3. Tragbare Vorrichtung (10) nach Anspruch 2, wobei die Vielzahl von ersten Belüftungslöchern (321) an Positionen angeordnet sind, die der Vielzahl von zweiten Belüftungslöchern (341) entsprechen.

4. Tragbare Vorrichtung (10) nach Anspruch 2, wobei die Patch-Einheit (200) ferner eine Stromstoppschicht (350) zwischen der Absorptionsschicht (330) und der zweiten Befestigungsschicht (340) aufweist, wobei die Stromstoppschicht (350) ein feuchtigkeitsdurchlässiges Material aufweist.

5. Tragbare Vorrichtung (10) nach Anspruch 1, die ferner eine Befestigungseinheit (400) mit einer Fläche umfasst, die größer als eine Fläche der Elektrodeneinheit (100) ist, wobei sich die Befestigungseinheit (400) auf der Elektrodeneinheit (200) befindet, sodass mindestens ein Teil davon an der Haut des Benutzers befestigt ist, und ein feuchtigkeitsdurchlässiges Material aufweist.

6. Verfahren zur Herstellung der tragbaren Vorrichtung (10) nach einem der Ansprüche 1 bis 5, wobei das Verfahren Folgendes umfasst:
Zusammenbauen der Patch-Einheit (300) durch Stapeln der Vielzahl von Schichten,
Stapeln der zusammengebauten Patch-Einheit (300), der Elektrodeneinheit (200) und der Hauptköpereinheit (100), und
Ausbilden der Vielzahl von Belüftungslöchern in der Patch-Einheit (300) durch Ausstanzen der Vielzahl von gestapelten Schichten unter Verwendung eines Presswerkzeugs.

## Revendications

1. Dispositif portable (10) configuré pour être utilisé en étant fixé à la peau d'un utilisateur, le dispositif portable (10) comprenant :
une unité de corps principale (100) incluant un logement (110) et une batterie (120), la batterie (120) étant disposée à l'intérieur du logement (110) ;
une unité d'électrodes (200) incluant une pluralité d'électrodes (221) connectées à la batterie (120) ; et
une unité de patch (300) incluant un ou plusieurs éléments conducteurs (310), l'un ou plusieurs éléments conducteurs (310) étant configurés pour connecter électriquement l'unité d'électrodes (200) à la peau de l'utilisateur, où l'unité de patch (100) inclut une pluralité de couches empilées, et une couche en contact avec la peau de l'utilisateur inclut une pluralité de trous de ventilation,
où l'unité d'électrodes (200) inclut en outre une couche de protection d'électrode (230) sur l'unité de patch (300), la couche de protection d'électrode (230) incluant, sur une partie intérieure dans le sens de la longueur de celle-ci, une rainure de montage (231) correspondant à une forme de l'unité de corps principale (100), et incluant, sur une partie extérieure dans le sens de la longueur de celle-ci, une pluralité de trous de ventilation (232) disposés selon un certain motif,
où l'unité d'électrodes (200) inclut en outre une couche de protection (210) disposée sous la couche de protection d'électrode (230), la couche de protection (210) incluant une pluralité de trous de ventilation (211) disposés selon un certain motif, à laquelle couche de protection un certain potentiel est appliqué par l'intermédiaire d'un circuit séparé de l'unité de corps principale (100), et
où les trous d'aération de la couche de protection d'électrode (232) sont disposés selon le même motif que la pluralité de trous d'aération (211) de la couche de protection (210), les formes de la pluralité de trous d'aération sont rectangulaires, et une largeur (W) d'un trou d'aération est supérieure à une distance (D) entre des trous d'aération adjacents.

2. Dispositif portable (10) selon la revendication 1, où l'unité de patch (300) inclut :
une première couche de fixation (320) en contact direct avec la peau de l'utilisateur, la première couche de fixation (320) incluant une pluralité de premiers trous de ventilation (321) ;
une couche d'absorption (330) sur la première couche de fixation (320) ; et
une deuxième couche de fixation (340) sur la couche d'absorption (330), la deuxième couche de fixation (340) étant configurée pour relier l'unité de patch (200) à l'unité de corps principale (100) et incluant une pluralité de deuxièmes trous de ventilation (341).

3. Dispositif portable (10) selon la revendication 2, où la pluralité de premiers trous de ventilation (321) sont disposés à des positions correspondant à la pluralité de deuxièmes trous de ventilation (341).

4. Dispositif portable (10) selon la revendication 2, où l'unité de patch (200) inclut en outre une couche d'arrêt de courant (350) entre la couche d'absorption (330) et la deuxième couche de fixation (340), la couche d'arrêt de courant (350) incluant un matériau perméable à l'humidité.

5. Dispositif portable (10) selon la revendication 1, comprenant en outre une unité de fixation (400) ayant une surface supérieure à la surface de l'unité d'électrodes (100), l'unité de fixation (400) étant sur l'unité d'électrodes (200) de telle sorte qu'au moins une partie de celle-ci soit fixée à la peau de l'utilisateur, et incluant un matériau perméable à l'humidité.

6. Méthode de fabrication du dispositif portable (10) selon l'une quelconque des revendications 1 à 5, la méthode comprenant :
assembler l'unité de patch (300) en empilant la pluralité de couches,
empiler l'unité de patch assemblée (300), l'unité d'électrodes (200) et l'unité de corps principale (100), et
former la pluralité de trous de ventilation dans l'unité de patch (300) en découpant la pluralité de couches empilées à l'aide d'un outil de presse.
